# FASCICULE DE BREVET EUROPEEN

(11) **EP 0 459 914 B1**
(45) Date de publication et mention de la délivrance du brevet: **03.08.1994**
(21) Numéro de dépôt: 91401424.6
(22) Date de dépôt: 30.05.1991
(51) Int. Cl.: A61F 2/08

(54) **Nouvel implant artificiel de ligament**
Implantat für einen künstlichen Bänderersatz
Artificial ligament implant

(30) Priorité: 01.06.1990 FR 9007042
(43) Date de publication de la demande: 04.12.1991
(73) Titulaire: Coisy, Michel, F-44230 Saint Sébastien Sur Loire (FR)
(72) Inventeur: Coisy, Michel, F-44230 Saint Sébastien Sur Loire (FR)
(74) Mandataire: Le Brusque, Maurice

(56) Documents cités:
- EP-A- 0 126 520
- EP-A- 0 170 358
- FR-A- 2 135 825
- FR-A- 2 278 313
- US-A- 4 255 820

## Description

La présente invention a trait au domaine chirurgical et plus particulièrement au domaine de la chirurgie ligamentaire ; elle concerne un nouvel implant artificiel de ligament, notamment destiné au remplacement des ligaments croisés antérieurs ou postérieurs du genou comme défini dans le préambule de la revendication 1. Un tel implant est connu du document US-A-4255820.

Il existe de très nombreux types de ligaments artificiels mis au point pour la réparation notamment des ligaments croisés du genou.

Ces ligaments sont généralement étudiés en fonction d'une technique opératoire préconisée et ils privilégient bien souvent soit les caractéristiques techniques intra-articulaires, soit les caractéristiques d'ancrage sur l'os récepteur.

On connaît par exemple du document EP-0126520 un ligament artificiel constitué d'une bande poreuse, tissée, ayant une structure homogène sur sa longueur. Cette structure poreuse permet de favoriser la réhabitation osseuse au niveau de l'ancrage de l'implant et cet ancrage est donc amélioré dans le temps ; les zones intra-articulaires et canalaires acceptent également une réhabitation tissulaire mais les propriétés mécaniques intra-articulaires, liées à la structure poreuse lâche du ligament peuvent, dans certains cas, ne pas être suffisantes.

Le document GB-2 159 846 présente un autre type d'implant de ligament constitué d'un assemblage de filaments en fibres de carbone dont les extrémités sont destinées à être solidarisées à l'os par l'intermédiaire de moyens d'ancrage tels que des vis. Ce type d'implant présente des caractéristiques mécaniques de résistance adaptées à sa fonction mais peut, à l'usage, présenter des inconvénients liés au bris, recul ou migration des moyens d'ancrage sur l'os. En outre, la structure ligamentaire vieillit dans le temps et risque de se dégrader progressivement.

On connaît encore par le document US-4 255 820 un implant de ligament obtenu à partir de fibres de polyester, conformé selon une structure tubulaire munie d'extrémités évasées.

Cet implant présente une densité poreuse croissante du centre vers les extrémités de façon à contrôler la réhabitation tissulaire biologique sur sa longueur. La structure est en particulier réalisée de telle sorte que les extrémités permettent une réhabitation fibreuse importante, en vue de fixer l'implant sur l'os ; à l'inverse, la partie centrale intra-articulaire est structurée et adaptée pour comparativement empêcher une réhabitation tissulaire. Là encore, le vieillissement du matériau ligamentaire diminue sa résistance dans le temps et augmente les risques de rupture.

L'invention a pour but de proposer un nouvel implant artificiel de ligament de conception très simple, ayant de bonnes propriétés mécaniques intra-articulaires et qui présente des caractéristiques qui permettent une réhabitation tissulaire intra-articulaire, une réhabitation osseuse rapide dans le ou les canaux osseux, ainsi qu'une fixation biologique sur l'os par réhabitation fibreuse extra-osseuse.

L'implant artificiel de ligament, selon l'invention, est décrit dans la revendication 1.

Le ligament artificiel ainsi obtenu n'est pas homogène sur sa longueur ; ses différentes zones sont adaptées selon leurs fonctions au niveau de l'articulation. Sa structure centrale renforcée accepte une réhabitation tissulaire, in situ, susceptible de pallier le vieillissement inévitable du matériau inerte support.

Selon une forme de réalisation préférée, l'implant artificiel est constitué d'une bande tissée à structure aérée, munie d'un renfort central solidarisé à la bande tissée support au moyen d'un surjet.

Selon une autre disposition de l'invention, la bande tissée et son renfort sont réalisés par tissage de fibres longitudinales et transversales de téréphtalate de polyéthylène glycol ; le tissage de chaque partie est homogène et est réalisé de telle sorte que les perforations aient une surface de l'ordre de 0,05 à 0,2 cm². La structure particulière de ce ligament et la porosité du tissage sont adaptées pour favoriser la croissance de tissus cellulaires à tous niveaux.

Toujours selon l'invention, le renfort central de l'implant est disposé sur toute la zone intra-articulaire et au moins sur une partie de la zone canalaire osseuse. Cette caractéristique évite le cisaillement lié à l'usure par frottement au niveau de l'entrée des canaux. Dans un mode de réalisation particulier, ce renfort sera présent sur l'ensemble de la partie intra-articulaire et de la partie osseuse. Pour assurer convenablement sa fonction, cette partie centrale renforcée s'étend en longueur sur 30 à 100 mm environ

Selon un mode de réalisation préféré, la partie centrale du ligament artificiel est enroulée sur elle-même, autour du renfort rapporté ; les bordures latérales de la bande, à ce niveau, sont assemblées au moyen d'un surjet.

Selon une autre caractéristique, la bande tissée et/ou son renfort consiste en une structure composite obtenue par tissage de fibres de collagène synthétique.

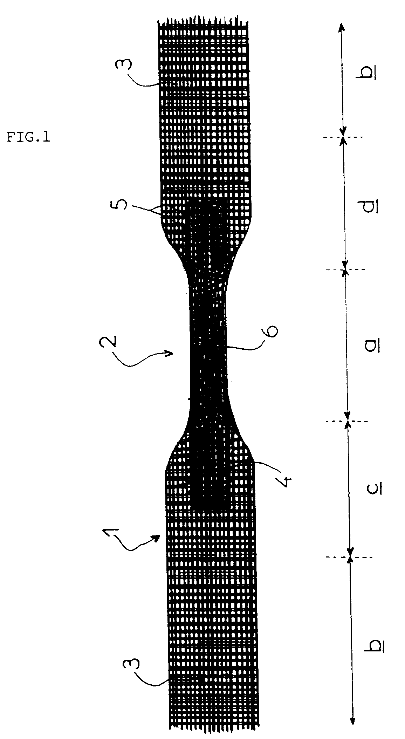

Mais l'invention sera encore illustrée sans être aucunement limitée par la description suivante d'un mode de réalisation particulier, donné à titre d'exemple et représenté sur le dessin annexé.

Tel qu'on l'a représenté, le ligament artificiel est réalisé à partir d'une structure allongée 1 en forme de bande ayant une longueur de l'ordre de 20 à 35 cm et une largeur de l'ordre de 1 à 3 cm.

Cette bande 1, aérée et lâche, présente une structure voisine de la gaze ; elle est obtenue de préférence par tissage de fibres longitudinales et transversales de téréphtalate de polyéthylène glycol. Le tissage des fibres est réalisé de façon à obtenir des résistances en traction adaptées à l'utilisation, de l'ordre de 1500 à 4000 newtons. Le tissage de la bande 1 est également réalisé de façon sufffisamment lâche pour obtenir une structure homogène aérée sur toute sa longueur, munie de perforations de l'ordre de 0,05 cm² à 0,2 cm².

La bande tissée 1 peut être obtenue en double épaisseur par pliage longitudinal d'une bande primaire de largeur double.

Le ligament artificiel selon l'invention comprend une partie centrale 2 et deux extrémités 3. La partie centrale 2 est munie de moyens de renfort qui permettent d'améliorer les propriétés mécaniques de l'implant à ce niveau. Dans l'exemple représenté, ces moyens de renfort consistent en une bande tissée 4, de même nature que la bande 1, rapportée et solidarisée sur ladite bande 1 au moyen d'un surjet périphérique 5. Ce surjet peut éventuellement n'être présent qu'au niveau des extrémités de la bande 4 ; il sera de préférence réalisé avec un fil de même nature que les bandes 1 et 4, c'est-à-dire un fil de téréphtalate de polyéthylène glycol. Le renfort 4 est centré sur la bande 1 ; il peut avoir une longueur de l'ordre de 30 à 100 mm et une largeur de l'ordre de 1 cm.

A titre indicatif, pour le remplacement d'un ligament croisé antérieur du genou, chez l'homme, on peut prévoir une bande support 1 ayant une résistance en traction de l'ordre de 3000 newtons, associée à un renfort 4 de 2450 newtons. La résistance efficace du ligament obtenu par l'association bande-renfort est alors de l'ordre de 5000 newtons ; la résistance extra-articulaire, au niveau des extrémités, est voisine de 3000 newtons. Dans un autre exemple de réalisation, une bande primaire 1 de 3000 newtons peut être associée à un renfort central 4 de 1450 newtons ; la résistance efficace du ligament est alors de l'ordre de 4000 newtons.

Pour compléter le renfort de la partie centrale 2 de l'implant artificiel, les bordures latérales de la bande 1 peuvent être, à ce niveau, enroulées sur elles-mêmes et assemblées au moyen d'un surjet 6. Ce surjet qui peut s'étendre sur une longueur de 30 à 100 mm permet de conférer une structure sensiblement tubulaire à la partie centrale 2 de l'implant, laquelle structure tubulaire renferme une partie au moins du renfort central 4.

Ce ligament prothétique peut avantageusement être utilisé pour le remplacement de ligaments croisés antérieurs ou postérieurs du genou, chez l'homme, ou du grasset, chez le chien, selon la résistance mécanique en traction désirée.

L'implantation d'un tel ligament artificiel au niveau du genou peut notamment être réalisée selon deux techniques opératoires : la technique en trans-condylien ou la technique en supra-condylien.

Dans les deux cas, la zone a, dite centrale, intra-articulaire est destinée à relier les deux os et notamment, dans le cas présent, le fémur et le tibia ; elle s'étend sur une longueur de l'ordre de 3 à 4 cm. Les deux zones b d'extrémité, qui correspondent au repère 3 de la bande 1, sont destinées à être ancrées sur l'os, par exemple par l'intermédiaire d'agrafes. Le surplus d'extrémité est éliminé par le chirurgien et de préférence étalé sur la partie corticale pour améliorer la réhabitation fibreuse extra articulaire.

La zone intermédiaire osseuse c qui s'étend d'un côté de l'implant, entre la zone centrale a et l'une des zones d'extrémité b, est destinée à se localiser, de façon classique, dans le canal tibial. Ce canal a généralement un diamètre de l'ordre de 5 mm.

La zone intermédiaire osseuse d qui s'étend de l'autre côté de l'implant, entre la zone centrale a et la zone d'extrémité opposée b, est destinée, selon la technique opératoire utilisée, à pénétrer dans un canal osseux réalisé dans le fémur, pour la technique trans-condylienne, ou à passer derrière le condyle dans le cas d'une technique supra-condylienne.

L'implant artificiel selon l'invention est de préférence réalisé par association de deux éléments 1, 4 homogènes. Cette association définit une structure non homogène sur sa longueur : aérée et lâche à ses extrémités, pour permettre une bonne réhabitation fibreuse extra-articulaire, au niveau de l'ancrage sur l'os, et plus dense dans sa partie centrale pour améliorer les propriétés mécaniques et en particulier la résistance dans la partie fonctionnelle intra-articulaire.

La structure aérée d'extrémité est apte à favoriser la croissance de tissu cellulaire autour des fibres de l'implant. Cette réhabitation fibreuse permet, si besoin, à terme, de s'affranchir des agrafes de fixation qui servent alors uniquement de moyens d'ancrage à efficacité temporaire. Etant donné ses trajets au niveau de l'articulation, les extrémités du ligament prothétique ne nécessitent pas une résistance mécanique aussi élevée que la partie centrale et on peut donc adapter les différentes parties de l'implant selon leurs fonctions au niveau de l'articulation. De part et d'autre du renfort central, les extrémités du ligament conservent une aération ou porosité constante.

Le renfort central est destiné à former au moins la partie intra-articulaire de l'implant ; il est constitué d'une structure plus dense qu'aux extrémités, en conservant une porosité constante sur sa longueur. Ce renfort s'étend au moins sur la longueur de la zone a et déborde avantageusement de part et d'autre de cette zone, sur au moins une partie des zones c et/ou d destinées à se positionner dans le ou les canaux osseux. Ce débordement permet de renforcer les parties ligamentaires au niveau de l'entrée dans les canaux osseux et limite les risques de cisaillement dans ces endroits sensibles.

La partie intra-articulaire de l'implant et les parties destinées à être incorporées dans le ou les canaux osseux acceptent, de par leur structure poreuse, une réhabitation tissulaire. Cette réhabitation va accélérer le processus de fixation biologique du ligament dans les zones canalaires et permet le renforcement de la zone intra-articulaire. Ce renforcement biologique intra-articulaire est destiné à pallier le vieillissement et l'affaiblissement inévitable du matériau inerte implanté ; on limite ainsi la perte de résistance dans le temps ce qui permet d'améliorer les propriétés de durabilité de la prothèse.

On peut noter en outre que la structure tissée, constituée de fibres longitudinales et transversales de matériau, limite l'élasticité du ligament uniquement à l'élasticité en longueur des fibres utilisées.

Bien entendu, l'utilisation de matériau autre que le téréphtalate de polyéthylène glycol peut être envisagée, que ce soit pour la bande support et/ou son renfort. On peut par exemple prévoir l'utilisation d'une structure composite obtenue par tissage de fibres de collagène synthétique ayant une résistance adaptée.

Les signes de référence insérés après les caractéristiques techniques mentionnées dans les revendications ont pour seul but de faciliter la compréhension de ces dernières et n'en limitent aucunement la portée.

## Revendications

1. Implant artificiel de ligament, notamment de ligament croisé du genou, lequel implant est en forme de bande tissée (1) à structure aérée, comportant une partie centrale (2) et deux extrémités (3), lesdites extrémités (3) étant destinées à être fixées sur un os et ladite partie centrale (2) étant renforcée sur au moins la partie intra-articulaire (a) de l'implant au moyen d'un renfort rapporté (4), caractérisé en ce que ladite bande tissée (1) a une structure homogène aérée sur toute sa longueur, ledit renfort rapporté (4) ayant une structure aérée voisine de celle de ladite bande (1) de façon à obtenir, par la combinaison de ladite bande et dudit renfort, une partie renforcée plus dense que les extrémités pour améliorer les propriétés mécaniques, tout en conservant une structure poreuse apte à permettre une réhabitation tissulaire.

2. Implant artificiel de ligament selon la revendication 1, caractérisé en ce qu'il est constitué d'une bande tissée (1), à structure aérée, munie d'un renfort central (4) solidarisé à ladite bande (1) par un surjet (5).

3. Implant artificiel de ligament selon la revendication 2, caractérisé en ce que le renfort central (4) est de même nature que la bande support (1).

4. Implant artificiel de ligament selon l'une quelconque des revendications 1 à 3, caractérisé en ce que le tissage de la bande (1) et de son renfort (4) est homogène et tel que les perforations aient une surface de l'ordre de 0,05 à 0,2 cm².

5. Implant artificiel de ligament selon l'une quelconque des revendications 1 à 4, caractérisé en ce que ladite partie centrale (2) est renforcée au niveau de la zone intra-articulaire (a) et au moins sur une partie des zones canalaires osseuses (c, d).

6. Implant artificiel de ligament selon la revendication 5, caractérisé en ce qu'il comporte un renfort (4) sur l'ensemble de la partie intra-articulaire (a) et de la ou ou des parties osseuses (c, d).

7. Implant artificiel de ligament selon l'une quelconque des revendications 1 à 6, caractérisé en ce qu'il comporte une partie centrale (2) renforcée sur 30 à 100 mm.

8. Implant artificiel de ligament selon l'une quelconque des revendications 1 à 7, caractérisé en ce qu'il comporte une partie centrale (2) enroulée sur elle-même, les bordures latérales de la bande (1), à ce niveau, étant assemblées au moyen d'un surjet (6).

9. Implant artificiel de ligament selon l'une quelconque des revendications 1 à 8, caractérisé en ce que la bande support (1) et son renfort (4) sont réalisés par tissage de fibres de téréphtalate de polyéthylène glycol.

10. Implant artificiel de ligament selon l'une quelconque des revendications 1 à 8, caractérisé en ce que la bande support (1) et/ou son renfort (4) consiste en une structure composite obtenue par tissage de fibres de collagène synthétique.

## Patentansprüche

1. Implantat für einen künstlichen Bänderersatz, insbesondere für Zwischenknochenkreuzbänder des Knies, wobei das Implantat in Form eines gewebten Bandes (1) mit lockerer Struktur vorliegt, das folgendes umfaßt: einen Mittelteil (2) und zwei Enden (3), wobei die Enden (3) dafür bestimmt sind, an einem Knochen befestigt zu werden, und wobei der Mittelteil (2) wenigstens im intraarticularen Teil (a) des Implantats mittels einer angefügten Verstärkung (4) verstärkt ist, dadurch gekennzeichnet, daß das gewebte Band (1) in seiner ganzen Länge eine homogene lockere Struktur hat, wobei die angefügte Verstärkung (4) eine lockere Struktur hat, die der des Bandes (1) ähnlich ist, so daß man durch Kombination des Bandes mit der Verstärkung einen verstärkten Teil erhält, der dichter als die Enden ist, um die mechanischen Eigenschaften zu verbessern, wobei zugleich eine poröse Struktur beibehalten wird, die eine Wiederansiedlung des Gewebes ermöglicht.

2. Implantat für einen künstlichen Bänderersatz gemäß Anspruch 1, dadurch gekennzeichnet, daß es aus einem gewebten Band (1) mit lockerer Struktur besteht, das mit einer Mittelverstärkung (4) ausgestattet ist, die durch eine überwendliche Naht (5) mit dem Band (1) fest verbunden ist.

3. Implantat für einen künstlichen Bänderersatz gemäß Anspruch 2, dadurch gekennzeichnet, daß die Mittelverstärkung (4) derselben Natur ist wie das Trägerband (1).

4. Implantat für einen künstlichen Bänderersatz gemäß einem der Ansprüche 1 bis 3, dadurch gekennzeichnet, daß das Band (1) und seine Verstärkung (4) homogen gewebt sind und so gewebt sind, daß die Perforationen eine Oberfläche im Bereich 0,05 bis 0,2 cm² besitzen.

5. Implantat für einen künstlichen Bänderersatz gemäß einem der Ansprüche 1 bis 4, dadurch gekennzeichnet, daß der Mittelteil (2) auf der Höhe der intraarticularen Zone (a) und wenigstens auf einem Teil der Knochenkanalzonen (c, d) verstärkt ist.

6. Implantat für einen künstlichen Bänderersatz gemäß Anspruch 5, dadurch gekennzeichnet, daß es eine Verstärkung (4) auf dem gesamten intraarticularen Teil (a) und dem (den) Knochenteil(en) (c, d) umfaßt.

7. Implantat für einen künstlichen Bänderersatz gemäß einem der Ansprüche 1 bis 6, dadurch gekennzeichnet, daß es einen Mittelteil (2) umfaßt, der über eine Länge von 30 bis 100 mm verstärkt ist.

8. Implantat für einen künstlichen Bänderersatz gemäß einem der Ansprüche 1 bis 7, dadurch gekennzeichnet, daß es einen Mittelteil (2) umfaßt, der auf sich selbst eingerollt ist, dadurch daß die Seitenränder des Bandes (1) auf dieser Höhe mit Hilfe einer überwendlichen Naht (6) miteinander verbunden sind.

9. Implantat für einen künstlichen Bänderersatz gemäß einem der Ansprüche 1 bis 8, dadurch gekennzeichnet, daß das Trägerband (1) und seine Verstärkung (4) durch Verweben von Fasern aus Polyethylenglycolterephthalat gefertigt sind.

10. Implantat für einen künstlichen Bänderersatz gemäß einem der Ansprüche 1 bis 8, dadurch gekennzeichnet, daß das Trägerband (1) und/oder seine Verstärkung (4) aus einer Kompositstruktur besteht, die durch Verweben synthetischer Kollagenfasern erhalten wurde.

## Claims

1. Artificial ligament implant, particularly crossed ligament of knee, said implant being an open weave strip (1) comprising a central part (2) and two ends (3), said ends (3) being provided to be fixed on a bone and said central part (2) being reinforced at least on the intra-articular part (a) of the implant by means of a joining reinforcement (4), characterized in that said woven strip (1) comprises on the whole length thereof a homogeneous open weave structure, said joining reinforcement (4) having an open structure close to the one of said strip (1) in order to obtain by the combination of said strip and said reinforcement a reinforced part more dense than the ends in order to improve the mechanical properties while having a porous structure liable to allow a tissular rehabilitation.

2. Artificial ligament implant according to claim 1, characterized in that it comprises an open weave strip (1) provided with a central reinforcement (4) linked to said strip (1) by a seam (5).

3. Artificial ligament implant according to claim 2, characterized in that the central reinforcement (4) is of the same type as the support strip (1).

4. Artificial ligament implant according to any one of claims 1 to 3, characterized in that the weaving of the strip (1) and the reinforcement (4) thereof is homogeneous and such that the open areas have an opening from 0.05 to 0.2 cm².

5. Artificial ligament implant according to any one of claims 1 to 4, characterized in that said central part (2) is reinforced at the level of the intra-articular area (a) and at least on one part of the bone canal areas (c, d).

6. Artificial ligament implant according to claim 5, characterized in that it comprises a reinforcement (4) on the whole intra-articular part (a) and on the bone part(s) (c, d).

7. Artificial ligament implant according to any one of claims 1 to 6, characterized in that it comprises a central part (2) reinforced on 30 to 100 mm.

8. Artificial ligament implant according to any one of claims 1 to 7, characterized in that it comprises a central part (2) wrapped on itself, the lateral wedges of the strip (1) at this level being assembled by means of a seam (6).

9. Artificial ligament implant according to any one of claims 1 to 8, characterized in that the support strip (1) and the reinforcement thereof are made by weaving polyethylene-glycol terephthalate fibers.

10. Artificial ligament implant according to any one of claims 1 to 8, characterized in that the support strip (1) and/or the reinforcement thereof comprise a composite structure obtained by weaving synthetic collagen fibers.
